# EUROPEAN PATENT SPECIFICATION

(11) **EP 3 831 351 B1**
(45) Date of publication and mention of the grant of the patent: **09.10.2024**
(21) Application number: 19843430.0
(22) Date of filing: 29.07.2019
(51) Int. Cl.: A61F 13/475

(54) **ABSORBENT ARTICLE**
ABSORBIERENDER ARTIKEL
ARTICLE ABSORBANT

(30) Priority: 30.07.2018 JP 2018142169
(43) Date of publication of application: 09.06.2021
(73) Proprietor: DAIO PAPER CORPORATION, Shikokuchuo-shi Ehime 799-0492 (JP)
(72) Inventor: YOSHIBA, Megumi, Sakura-shi, Tochigi 329-1411 (JP)
(74) Representative: Schön, Christoph
(86) International application number: PCT/JP2019/029653
(87) International publication number: WO 2020/027051

(56) References cited:
- WO-A1-2018/101192
- JP-A- 2009 011 685
- JP-A- 2009 011 685
- JP-A- 2017 104 436
- US-A- 5 387 210
- US-A1- 2010 168 707

## Description

### Technical Field

The present invention relates to an absorbent article to be used in incontinence pads, panty liners, sanitary napkins, and the like, and, in particular, to an absorbent article having a pair of left and right side sheets on both side portions of a skin-contact surface side.

### Background Art

Conventionally, as the absorbent article, there is known an absorbent article including an impermeable back-surface sheet of a polyethylene sheet, a polyethylene sheet-laminated nonwoven fabric, or the like, a top-surface sheet, and an absorber made of paper cotton such as pulverized pulp interposed between the back-surface sheet and the top-surface sheet, and including a pair of left and right side sheets over an entire length of the both side portions on a skin-contact surface side.

The side sheets are disposed to have an overlapping allowance in which the side sheets overlap with the top-surface sheet in the thickness direction on an inner side in a width direction on the skin-contacting surface side of the absorbent article. The top-surface sheet is joined to the overlapped portion by a joining portion that has been formed in various forms. As the joining, for example, Patent Document 1 discloses that a plurality of embossed portions having compressed portions formed along the side sheets is provided, wherein the embossed portions include a first embossed portion formed in both edge portions, that is, a front edge side and a back edge side, in a longitudinal direction of the absorbent article, a second embossing portion formed in a central portion in the longitudinal direction, and the first embossed portion is wider in a width direction orthogonal to the longitudinal direction than the second embossed portion, and compressed portions constituting the second embossed portion are formed so as to be spaced apart from each other.

Furthermore, the Patent Document 2 discloses that a leakage-preventing unit formed of side sheets includes a central region, and a pair of end unit regions positioned at an outer side from the central region in the longitudinal direction, and that the end unit region includes a joint processing unit carrying out joint processing for joining portions which the side sheets face, and a non-joint processing unit in which joint processing is not carried out.

Furthermore, Patent Document 3 discloses that a compression part compressing and joining an inner end in the width direction of the side sheet to a top-surface sheet in the skin side of the surface sheet in a position overlapping with a side region in the width direction of the absorber is formed, and a cover sheet is provided to cover the compression part from the skin surface side and to make the compression part hard to be seen from the skin surface side of the surface sheet.
JP 2009-011685 A discloses an absorbent article comprising an absorptive main body provided with a liquid permeable surface layer, a liquid retentive absorption layer and a leakage prevention layer, and a pair of left and right side sheets provided over the almost entire length of both side edge parts on the skin abutting surface side of the absorptive main body, wherein first embosses formed only on the side sheets and second embosses formed so as to connect the side sheets and one of the surface layer, absorption layer and leakage prevention layer of the absorptive main body continuously or intermittently columnarly in the longitudinal direction of the side sheets are disposed.
US 5 387 210 A discloses a sanitary napkin with flaps including wings formed on laterally opposite sides thereof, wherein top surfaces of the flaps are formed by liquid barrier sheets of which the inner areas form together with the topsheet pockets therebetween to receive menstrual fluid, and wherein the pockets are opened as the wings are folded onto the outer sides of a crotch zone of a user's underpants to fix the napkin thereto.

### Citation List

### Patent Document

Patent Document 1: Japanese Unexamined Patent Application, Publication No. 2008-289535
Patent Document 2: Japanese Unexamined Patent Application, Publication No. 2010-264161
Patent Document 3: Japanese Unexamined Patent Application, Publication No. 2017-104436

### Summary of Invention

### Technical Problem

However, in the configurations described in the above Patent Documents 1 and 2, it has been pointed that since a joining portion for joining side sheets and a top-surface sheet is formed of embossed portions obtained by pressing from a skin-contact surface side of the side sheets, when an area of the embossed portions is increased in order to enhance the joining strength, touch to the skin is deteriorated, and meanwhile, when an area of the embossed portions is reduced in order to prevent the touch to the skin from being deteriorated, the joining strength is reduced and peeling occurs easily.

On the other hand, in the configuration described in the above Patent Document 3, a compression part compressed and joined to the top-surface sheet is provided on the inner-side end portion in the width direction of the side sheet, and a cover sheet for covering the compression part is coupled to the side sheet via only an adhesive layer. However, the adhesive layer has a problem that in low temperature conditions such as winter, adhesive property is deteriorated, and the adhesive layer is easily peeled off. When the adhesive layer is peeled off, twists or creases easily occur in a laminated portion of the side sheets, which may cause deterioration of wearing feeling. Furthermore, when an adhesive layer is used as joining means in a sheet material that comes into contact with skin surface, when the basis weight of the adhesive is increased in order to enhance the joining strength, the adhesive penetrates through the sheet and floats at the skin surface side, thus causing sticky feeling, and deteriorating wearing feeling.

Then, a main object of the present invention is to provide an absorbent article configured to suppress deterioration of touch to the skin and to obtain good wearing feeling while sufficient joining strength is maintained in the joining portion of the side sheets.

### Solution to Problem

In order to solve the problem mentioned above, the present invention provides an absorbent article as defined in appended claim 1

In the invention according to the claim 1, the inner side in the width direction of each of the side sheets is folded back in the width direction to be laminated in a plurality of layers, and thereby a laminate portion of the side sheet is formed. The laminate portion of the side sheet includes a lower layer side embossed portion for joining a bottom layer of the side sheet and the top-surface sheet to each other, and a skin-side embossed portion obtained by integrally denting the side sheets of the laminated portion from the skin-contact surface side. In this way, the lower layer side embossed portion is not brought into direct contact with the skin surface because the laminated portion of each of the side sheets is laminated on the skin-contact surface side. Therefore, even when the lower layer side embossed portion is formed in a large area having sufficient joining strength, deterioration of touch to the skin can be suppressed. On the other hand, the skin-side embossed portion formed at the skin-contact surface side in the laminated portion of the side sheet may be formed with joining strength sufficient to prevent the side sheets from being peeled off from each other in the laminated portion, and a sense of discomfort does not occur even when the side sheets are brought into direct contact with the skin surface. Therefore, good wearing feeling is achieved.

As the present invention according to claim 2, the absorbent article according to claim 1 in which the lower layer side embossed portions and the skin-side embossed portions are formed in different plane patterns is provided.

In the invention according to claim 2, the lower layer side embossed portions and the skin-side embossed portions are formed in different plane patterns such that the lower layer side embossed portions are formed in a plane pattern that places an importance on the joining strength with respect to the top-surface sheet, and the skin-side embossed portions are formed in a plane pattern that places an importance on the appearance seen from the skin-contact surface side of the absorbent article and improves decorativeness.

As the present invention according to claim 3, the absorbent article according to claim 1 or 2 in which the skin-side embossed portions are formed to have a smaller area of the embossed portions per unit area than the lower layer side embossed portions.

In the invention according to claim 3, the skin-side embossed portions are formed in a smaller area than that of the lower layer side embossed portions in order to reduce the bad skin contact when the skin-side embossed portions are brought into contact with the skin surface and to enhance the joining strength with the top-surface sheet by the lower layer side embossed portions.

As the present invention according to claim 4, the absorbent article according to any one of claims 1 to 3 is provided in which wherein the lower layer side embossed portions are formed in an intermittent plane pattern in which a pressed portion and a non-pressed portion are alternately disposed along a longitudinal direction of the absorbent article.

In the invention according to claim 4, the plane pattern of the lower layer side embossed portions is an intermittent pattern in which a pressed portion and a non-pressed portion are alternately disposed along a longitudinal direction of the absorbent article, and thereby the pressure at the time of being pressed is distributed so as to enhance joining strength between the side sheet and the top-surface sheet.

As the present invention according to claim 5, the absorbent article according to any one of claims 1 to 4 is provided in which the lower layer side embossed portions and the skin-side embossed portions are formed in regions overlapping with each other in a thickness direction.

The invention according to claim 5 is a first embodiment example showing a relative positional relationship between the lower layer side embossed portions and the skin-side embossed portions, in which the lower layer side embossed portions and the skin-side embossed portions are formed in regions overlapping with each other in a thickness direction. Thus, the lower layer side embossed portions can be formed in a wide range of the laminated portion of the side sheet, and the joining strength between the side sheet and the top-surface sheet can be reliably enhanced.

As the present invention according to claim 6, the absorbent article according to any one of claims 1 to 4 is provided in which the lower layer side embossed portions are formed in a region at an inner side in the width direction, and the skin-side embossed portions are formed in a region at an outer side in the width direction, and thereby the lower layer side embossed portions and the skin-side embossed portions are formed in regions that do not overlap with each other in the thickness direction.

The invention according to claim 6 is a second embodiment example showing a relative positional relationship between the lower layer side embossed portions and the skin-side embossed portions, and the lower layer side embossed portions and the skin-side embossed portions are formed in regions that do not overlap with each other in the thickness direction. By forming the lower layer side embossed portions in a region at an inner side in the width direction, the lower layer side embossed portions can be a base end for folding back the side sheet, and thus, the side sheet can be folded back easily. By forming the skin-side embossed portions in a region at an outer side in the width direction, the skin-side embossed portions are not formed at the inner side in the width direction, which is easily brought into contact with the skin surface. Thus, the skin-side embossed portions can further be prevented from being brought into contact with the skin surface, and the skin contact can further be made softer.

As the present invention according to claim 7, the absorbent article according to any one of claims 1 to 6 is provided in which each of the side sheets is disposed so as to extend toward the inner side portion in the width direction in a state in which one side portion is substantially aligned to the side portion of the top-surface sheet, and is folded back toward the skin-contact surface side from the lower layer side embossed portions as a base such that another side portion extends to the side portion of the absorbent article.

The absorbent article according to claim 7 is a preferable laminated state of the laminated portion of the side sheet, and has a two-layer structure folded back at the inner side in the width direction. In this way, the side sheet is folded back to the skin contact surface side at the inner side in the width direction, and the folded side portion is extended to the side portion of the absorbent article. Thus, the side portion of the side sheet is not brought into contact with the skin surface, so that the touch to the skin can be further satisfied.

As the present invention according to claim 8, the absorbent article according to any one of claims 1 to 7 is provided in which an elastically stretchable member is disposed between the layers of the side sheets laminated in a plurality of layers along the longitudinal direction of the absorbent article, and the elastically stretchable member is disposed at the inner side in the width direction from the lower layer side embossed portions and the skin-side embossed portions.

In the absorbent article according to claim 8, the elastically stretchable member is disposed between the layers of the laminated side sheets. By disposing the elastically stretchable member at the inner side in the width direction from the lower layer side embossed portions and the skin-side embossed portions, these embossed portions can exhibit functions of the elastically stretchable member without being restricted by the shrinkage force.

As the present invention according to claim 9, the absorbent article according to any one of claims 1 to 8 is provided in which the lower layer side embossed portions are formed to have a sectional shape bulging toward a skin-contact surface side.

In the invention according to claims 9, by forming the lower layer side embossed portions to have a cross sectional shape bulging toward the skin-contact surface side, the cushioning characteristic of the laminated portion of the side sheet is improved, and the skin contact can further be made softer.

### Advantageous Effect of Invention

As described above in detail, according to the present invention, the deterioration of touch to the skin can be suppressed and preferable wearing feeing can be obtained while sufficient joining strength is secured in the joining portion of the side sheet is secured.

### Brief Description of Drawings

FIG. 1 is a partially broken development view of a sanitary napkin 1 according to the present invention.
FIG. 2 is an arrow view taken along line II-II in FIG. 1.
FIG. 3 is an enlarged plan view of a laminated portion 8 of a side sheet 7.
FIG. 4 is an arrow view taken along line IV-IV in FIG. 3.
FIG. 5 is a plan view showing a lower layer side embossed portions 9.
FIG. 6 is a plan view showing a modified example of the lower layer side embossed portions 9.
FIG. 7 is a plan view showing the modified example of a skin-side embossed portions 10.
FIG. 8 is an enlarged plan view showing a laminated portion 8 of a side sheet 7 according to the modified example.
FIG. 9 is an arrow view taken along line IX-IX in FIG. 8.
FIG. 10 is a development view of a sanitary napkin 1 according to the modified example.
FIG. 11 is an arrow view taken along line XI-XI in FIG. 10.
FIG. 12 is a perspective view showing a process for processing an embossed portions.
FIG. 13 is a sectional view of a top-surface sheet 3 and a side sheet 7 according to another embodiment example.

### Description of Embodiments

Hereinafter, the present invention will be described in detail with reference to the drawings.

### [One Example of Basic Structure of Sanitary Napkin]

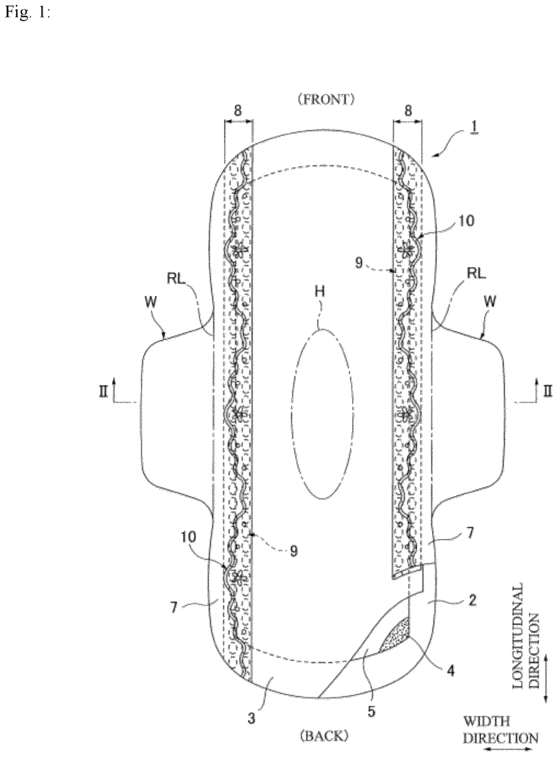

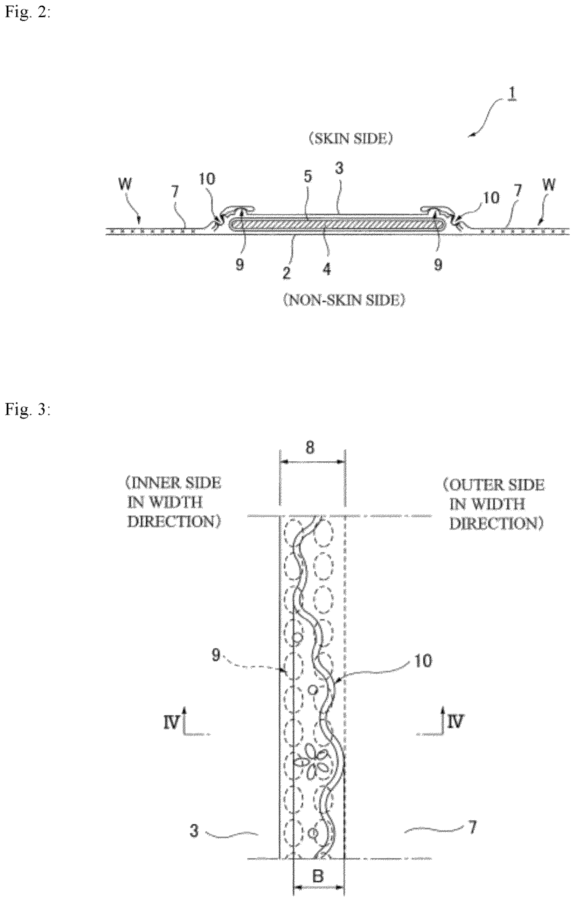

A sanitary napkin 1 according to the present invention mainly includes, as shown in FIGs. 1 and 2, an impermeable back-surface sheet 2 made of, for example, a polyethylene sheet; a top-surface sheet 3 forming a skin-contact surface and allowing rapid permeation of a body fluid; an absorber 4 interposed between the both sheets 2 and 3 and made of cotton pulp, synthetic pulp, or the like; an encapsulating sheet 5 covering at least a skin-side surface and a non-skin-side surface of the absorber 4 for shape retention and improvement of diffusivity of this absorber 4 and made of a crepe paper, a nonwoven fabric, or the like; and a pair of left and right side sheets 7 provided on both side portions of the skin-contact surface side over a substantially entire length of the sanitary napkin 1. In the surrounding of the absorber 4, at upper and lower end edge portions thereof, outer side edge portions of the back-surface sheet 2 and the top-surface sheet 3 are joined by joining means such as an adhesive such as hot melt, heat seal, an ultrasonic seal. Furthermore, at both side edge portions thereof, the back-surface sheet 2 and a side sheet 7 extending further to the lateral side than the absorber 4 are joined with joining means such as an adhesive such as a hot melt, a heat seal, and an ultrasonic seal. In an example shown in the drawings, the absorber 4 has a single-layer structure but may have a multi-layered structure forming a middle-height portion, and also may have a multi-layered structure in which absorbers having the same size and shape are stacked. Furthermore, as necessary, a hydrophilic second sheet may be interposed between the top-surface sheet 3 and absorber 4.

For the back-surface sheet 2, a sheet material having at least water-blocking properties, for example, polyethylene, is used. In recent years, however, from the viewpoint of preventing a moist feeling, a sheet material having moisture permeability has tended to be used. As the water-blocking and moisture-permeable sheet materials, microporous sheets are suitably used. A microporous sheet is obtained by molding a sheet by melting and kneading an inorganic filler in an olefin-based resin such as polyethylene or polypropylene, followed by stretching in uniaxial or biaxial direction. On a non-skin-contact surface side (outside surface) of the back-surface sheet 2, one or a plurality of stripes of sticking layers (not shown in the drawings) is formed, so that the sanitary napkin 1 is fixed to underwear when wearing on the body. As the back-surface sheet 2, a poly-laminate nonwoven fabric in which a plastic film and a nonwoven fabric are laminated may be used.

Next, for the top-surface sheet 3, porous or non-porous non-woven fabric, a porous plastic sheet, or the like, are suitably used. As the material fibers constituting the nonwoven fabric, synthetic fibers such as olefin-based fibers such as polyethylene or polypropylene, polyester-based fibers, and polyamide-based fibers, regenerated fibers such as rayon and cupra, and natural fibers such as cotton can be used, and nonwoven fabrics obtained by appropriate processing methods such as a spun lace method, a spun bond method, a thermal bond method, a meltblown method, and a needle punch method can be used. Among these processing methods, the spun lace method is excellent in being rich in flexibility, and a drape property, and the thermal bond method is excellent in bulkiness and high compression restorability. When a large number of through holes are formed in the top-surface sheet 3, the body fluid is rapidly absorbed, so that excellent dry touch property is achieved. The non-woven fabric fibers may be long fibers or short fibers, but short fibers are preferably used in order to bring out the texture of towel fabric. Furthermore, in order to fabricate embossing treatment, olefin-based fibers such as polyethylene or polypropylene having a relatively low melting point is preferably used. Furthermore, a core-sheath type fiber including a core having a high melting point and a sheath having a low melting point, a side-by-side fiber, or composite fiber of dividing fibers can be suitably used.

The absorber 4 can absorb and retain the body fluid, and the absorber 4 used can be one in which a particulate superabsorbent polymer is dispersed and mixed in fluffy pulp fiber, or a polymer sheet including an upper layer sheet at a skin-contact surface side, a lower layer sheet at a non-skin-contact surface side, and the highly absorbent polymer interposed between the upper layer sheet and the lower layer sheet.

Examples of the pulp fiber include a fiber made of cellulose fibers such as chemical pulp obtained from timber or molten pulp, and artificial cellulose fibers such as rayon and acetate. From the viewpoint of function and price, softwood pulp having a fiber length longer than that of hardwood pulp is suitably used. The basis weight of the pulp fiber is set at 150 to 700 g/m², and preferably 250 to 400 g/m². The basis weight of the superabsorbent polymer is set at 15 to 470 g/m², and preferably 15 to 100 g/m².

Examples of the superabsorbent polymer include crosslinked polyacrylate, self-crosslinked polyacrylate, a saponified product of a crosslinked product of an acrylic acid ester-vinyl acetate copolymer, a crosslinked product of an isobutylene/maleic anhydride copolymer, a crosslinked product of polysulfonate, and a partially crosslinked water-swelling polymer such as polyethylene oxide or polyacrylamide. Among these examples, acrylic acid or acrylate salt-based being excellent in absorption amount and water absorption speed is suitable. In the manufacturing process, an absorption ratio (water absorption power) and an absorption speed of the superabsorbent polymer having absorbing performance may be adjusted by adjusting a crosslink density and a crosslink density gradient.

### [Side sheet]

In the example shown in the drawings, the top-surface sheet 3 is formed in a width to an extent that the top-surface sheet 3 has a width slightly larger than a width of the absorber 4 and only covers the absorber 4. The outer side in the width direction of the top-surface sheet 3 is covered with the side sheet 7 (a member other than the top-surface sheet 3) extending from the skin-contact surfaces of both side portions of the top-surface sheet 3. As the side sheet 7, depending on objectives, for example, prevention of permeation of a body fluid or enhancement of touch to the skin, a nonwoven fabric material, to which appropriate water repelling treatment or hydrophilic treatment has been applied, can be used. As such side sheet 7, one formed by an appropriate processing method using a natural fiber, a synthetic fiber, or a regenerated fiber as a raw material can be used. However, in order to eliminate a stiff feeling and to prevent a moist feeling, a nonwoven fabric having aeration properties with the basis weight reduced is preferable. Specifically, it is desirable to use a nonwoven fabric produced by setting the basis weight at 8 to 23 g/m² is desirably used.

As shown in FIG. 2, the side sheet 7 is disposed over a range on the outer portion from a middle portion in the width direction of the sanitary napkin 1 from a predetermined inner position overlapping with the absorber 4 in the thickness direction by way of the side edge of the absorber to the outer periphery of the back-surface sheet 2. The laminate sheet portion of these side sheets 7 and back-surface sheet 2 forms a flap portion in which absorber 4 is not interposed at the both side portions of the absorber 4. As shown in FIG. 1, the flap portion forms a pair of left and right wing-like flaps W, W in a position of the side portions of a region substantially corresponding to a body fluid discharge portion H of a wearer. At the outer surface side of the wing-shaped flaps W, W, an adhesive layer (not shown) is provided. At the time of placing to the shorts, the wing-like flaps W, W are folded back to the non-skin contact surface side at the folding line RL position in the base end portion, and wound around the crotch part of the shorts and fixed.

On the other hand, the inner side in the width direction of the side sheet 7 is folded back in the width direction to form the laminated portion 8 of a plurality of layers. The laminated portion 8 of the side sheet 7 is formed over the entire length of the side sheet 7 at substantially equal width along the longitudinal direction of the sanitary napkin 1, and is formed in a range including a region overlapping with the top-surface sheet 3 in the thickness direction. In other words, at least a part of the inner side in the width direction of the laminated portion 8 has an overlapping allowance with the top-surface sheet 3 in the thickness direction, preferably an entire part of the laminated portion 8 overlaps with the top-surface sheet 3 in the thickness direction, and more preferably, the outer side edge portion in the width direction of the laminated portion 8 is substantially aligned to side edge portion of the surface sheet 3.

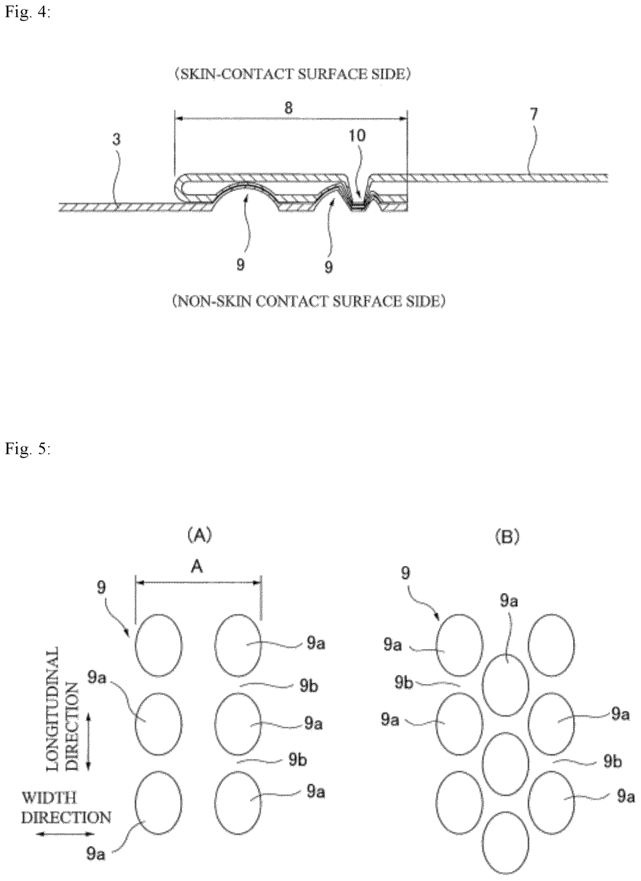

As shown in FIGs. 3 and 4, the laminated portion 8 of the side sheet 7 includes lower layer side embossed portions 9 for joining the bottom layer of the side sheet 7 and the top-surface sheet 3, and a skin-side embossed portions 10 obtained by integrally denting the side sheet 7 of the laminated portion 8 from the skin-contact surface side. The lower layer side embossed portions 9 are embossed portions for a structure for strongly joining the side sheet 7 and the top-surface sheet 3. The skin-side embossed portions 10 are embossed portions integrating the laminated side sheets 7, preventing the side sheets 7 from twisting and wrinkling in the laminated part 8, and including a decorative meaning for improving the appearance from the skin-contact surface side of the sanitary napkin 1.

The lower layer side embossed portions 9 and the skin-side embossed portions 10 are portions in which fibers are thermally fused by applying heat or ultrasonic waves at the time of pressing. In view of making the skin contact soft, at least the skin-side embossed portions 10 preferably uses ultrasonic waves as thermally fusing means.

In the embodiment example in FIG. 4, the side sheet 7 is disposed to extend toward the inner side in the width direction in a state in which one side portion is substantially aligned to the side portion of the top-surface sheet 3, and folded back toward the skin-contact surface side from the edge portion at the inner side in the width direction of the lower layer side embossed portions 9 as a base end, and another side portion extends toward the side portion of the sanitary napkin 1. Thus, in the embodiment example shown in FIG. 4, the laminated portion 8 is formed by a two-layer structure.

In this way, in the sanitary napkin 1, a sheet portion in the laminated portion 8 of the side sheet 7 in which the lower layer side embossed portions 9 are not provided is laminated at the skin-contact surface side of the lower layer side embossed portions 9, and thereby the lower layer side embossed portions 9 are not brought into direct contact with the skin when wearing. Therefore, even if the lower layer side embossed portions 9 are an embossed portion formed in a large area having a sufficient joining strength, the deterioration of touch to the skin can be suppressed. On the other hand, the skin-side embossed portions 10 formed on the skin-contact surface side of the laminated portion 8 is only required to be formed with a joining strength sufficient to prevent peeling of the side sheets 7 from each other in the laminated portion 8, uncomfortable feeling does not occur even when the side sheets 7 are brought into direct contact with the skin surface. Therefore, excellent wearing feeling is obtained.

Furthermore, since the skin-side embossed portions 10 can prevent peeling of the side sheets 7, 7 from each other in the laminated portion 8, twisting and wrinkling do not easily occur in the side sheet 7 of the laminated portion 8. Furthermore, by forming the skin-side embossed part 10 in a wavy plane pattern or adding a pattern such as a flower pattern, the appearance of the absorbent article seen from the skin-contact surface side becomes good, and the decorativeness can be improved.

As shown in FIG. 3, it is preferable that the lower layer side embossed portions 9 and the skin-side embossed portions 10 are formed in different plane patterns. In other words, the lower layer side embossed portions 9 are preferably formed in a plane pattern that can strongly join the side sheet 7 and the top-surface sheet 3 to each other. The skin-side embossed portions 10 are preferably formed in a plane pattern that does not easily deteriorate the touch to the skin and that is excellent in decorativeness.

In order not to deteriorate the touch to the skin when wearing, it is preferable that an area of the embossed portions per unit area of the skin-side embossed portions 10 is smaller than that of the lower layer side embossed portions 9. The area of the embossed portions per unit area is a value obtained by dividing a total area of the lower layer side embossed portions 9 or a total area of the skin-side embossed portions 10 by an area of the laminated portion 8. When the skin-side embossed portions 10 are formed to have a smaller area than the area of the lower layer side embossed portions 9, the deterioration of touch to the skin can be suppressed while the sufficient joining strength of the side sheet 7 and the top-surface sheet 3 is ensured by the lower layer side embossed portions 9. As the rate of the area, the area of the skin-side embossed portions 10 is preferably 1/2 to 1/50, and preferably 1/5 to 1/20 with respect to the area of the lower layer side embossed portions 9.

As shown in FIG. 5, it is preferable that the lower layer side embossed portions 9 are formed in an intermittent plane pattern in which pressed portion 9a and the non-pressed portion 9b are positioned alternately along the longitudinal direction of the sanitary napkin 1. When the lower layer side embossed portions 9 are formed in an intermittent pattern, since pressure at the time of pressing the lower layer side embossed portions 9 are distributed, the joining strength between the side sheet 7 and the top-surface sheet 3 can be further enhanced. Furthermore, even when the side sheet 7 and the top-surface sheet 3 are strongly joined to each other with the lower layer side embossed portions 9, the sanitary napkin 1 can be deformed flexibly because it is easily folded at the non-pressed portion 9b. Therefore, the sanitary napkin 1 is easily fitted to the skin surface.

As shown in FIG. 5, the lower layer side embossed portions 9 can include one or a plurality of the pressed portions 9a arranged in the width direction of the sanitary napkin 1. In the example shown in the drawing, two pressed portions 9a, 9a are arranged in the width direction. As shown in the drawing, by arranging a plurality of embossed portions in the width direction, the joining strength between the side sheet 7 and the top-surface sheet 3 can be further enhanced. When the plurality of the pressed portions 9a is arranged in the width direction, adjacent pressed portions 9a can be arranged in regular lattice in which the adjacent pressed portions 9a are arranged in the longitudinal direction and the width direction as shown in FIG. 5(A), or can be arranged in a rhombic lattice shape in which the pressed portions 9a adjacent in the width direction are shifted by half pitch in the longitudinal direction as shown in FIG. 5(B). When the pressed portions 9a are arranged in a regular lattice, the laminated portion 8 can be easily bent along the roundness of the front and back of the body. When the laminated portions 8 are arranged in the diamond lattice shape, the laminated portion 8 can be easily deformed in the oblique direction.

Furthermore, as shown in FIG. 5, a provided width A of the lower layer side embossed portions 9 may be any width as long as it is within the width of the laminated portion 8, but it is preferably 3 to 15 mm, and more preferably 5 to 10 mm.

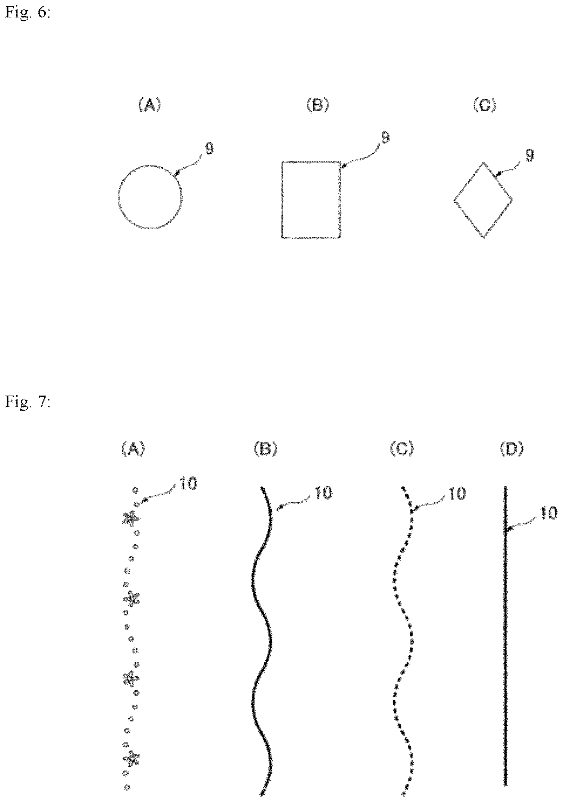

A planar shape of the pressed portion 9a of the lower layer side embossed portions 9 in the example shown in FIG. 5 is an elliptical shape that is longer in the longitudinal direction of the sanitary napkin 1, but the pressed portion 9a may be formed in any shapes such as (A) a circle, (B) a rectangle, and (C) a rhombus as shown in FIG. 6.

The lower layer side embossed portions 9 can be formed by pressing from the outer surface side of the top-surface sheet 3 or the outer surface side of the side sheet 7 in a state in which the top-surface sheet 3 and the side sheet 7 are laminated. In other words, the pressing direction may be any directions, and furthermore, pressing may cause bulging toward the opposite side of the pressing or may not cause bulging. Preferable embodiment is a form in a dome-shaped cross sectional shape bulging toward the skin-contact surface side as shown in FIG. 4. Formation in a cross sectional shape bulging toward the skin-contact surface side increases the cushioning characteristic of the laminated portion 8, and further makes the skin contact in this portion softer.

Next, the skin-side embossed portions 10 are described. The skin-side embossed portions 10 may be formed continuously along substantially the longitudinal direction as shown in FIGs. 1 and 3, or may be formed intermittently in which a pressed portion and a non-pressed portion are alternately disposed as shown in FIGs. 7(A) and 7(C).

The skin-side embossed portions 10 preferably includes a wavy line, reciprocating in the width direction, along the substantially longitudinal direction of the sanitary napkin 1 as shown in FIGs. 1, 3, and 7(A) to 7(C). When the skin-side embossed portions 10 include a wavy line, the deformation of the laminated part 8 along the body when wearing is not easily inhibited, and decorativeness in appearance becomes excellent. Furthermore, in order to further improve the decorativeness, as shown in FIGs. 3 and 7(A), a pressed portion consisting of concrete shapes such as a flower pattern, a star shape and a heart shape, or a geometric shape such as a circle or a polygon may be discretely arranged on the wavy line or in the vicinity of the wavy line. On the other hand, the skin-side embossed portions 10 may be formed in a continuous line or an intermittent line along the longitudinal direction of the sanitary napkin 1 as shown in FIG. 7(D).

Furthermore, similar to the lower layer side embossed portions 9, the skin-side embossed portions 10 may include one or a plurality of intermittent patterns located intermittently along the longitudinal direction of the sanitary napkin 1, in the width direction of the sanitary napkin 1. In this case, the joining strength of the skin-side embossed portions 10 is preferably smaller than the joining strength of the lower layer side embossed portions 9. This can make the skin contact of the skin-side embossed portions 10 soft. The joining strength can be reduced by both or either means of reducing the pressing depth during pressing, and means of reducing the heating temperature.

Furthermore, as shown in FIG. 3, it is preferable that a providing width B of the skin-side embossed portions 10 is smaller than the providing width A of the lower layer side embossed portions 9. Specifically, it is 1 to 10 mm, and preferably 1 to 5 mm.

As shown in FIG. 3, the lower layer side embossed portions 9 and the skin-side embossed portions 10 can be formed in regions overlapping in the thickness direction. In other words, the lower layer side embossed portions 9 are formed in the entire of the laminated portion 8, and the skin-side embossed portions 10 are formed in a region overlapping with the lower layer side embossed portions 9 in the thickness direction. Thus, since the lower layer side embossed portions 9 can be formed in a wide range of the laminated portion 8, the joining strength between the side sheet 7 and the top-surface sheet 3 can be further enhanced. In FIG. 3, the skin-side embossed portions 10 are formed along substantially the entire width of the laminated portion 8, but in view of the fact that the inner side in the width direction of the laminated portion 8 is a region in which the laminated portion 8 is easily brought into contact with the skin surface, the skin-side embossed portions 10 may be disposed only in the outer range in the width direction of the laminated portion 8 in order to further soften the skin contact.

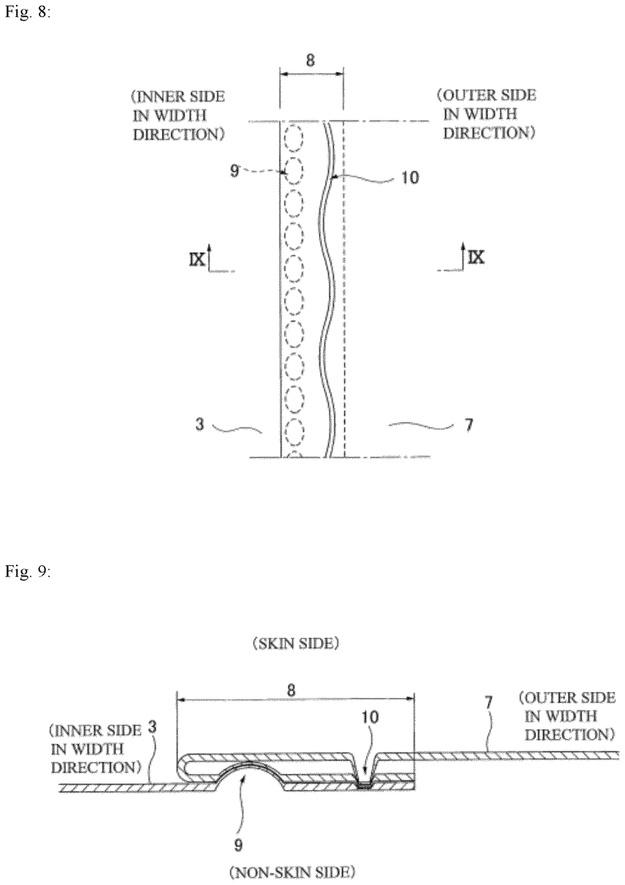

Furthermore, as a modified example, as shown in FIGs. 8 and 9, the lower layer side embossed portions 9 are formed in a region at the inner side the width direction, and the skin-side embossed portions 10 are formed in a region at the outer side in the width direction. Thus, the lower layer side embossed portions 9 and the skin-side embossed portions 10 can be formed in regions that do not overlap with each other in the thickness direction. When the lower layer side embossed portions 9 are formed in a region at the inner side in the width direction, the lower layer side embossed portions 9 are a base end of folding when the side sheet 7 is folded back in the manufacturing step, and the side sheet 7 is easily folded. When the skin-side embossed portions 10 are formed in a region at the outer side in the width direction, the skin-side embossed portions 10 are not formed in the region that tends to be brought into contact with the skin surface at the inner side in the width direction. Therefore the skin-side embossed portions 10 are not further easily brought into contact with the skin surface, and the skin contact can be made softer.

When the skin-side embossed portions 10 are formed in a region at the outer side in the width direction, the skin-side embossed portions 10 are disposed preferably at the outer side from the absorber 4. Thus, the skin-side embossed portions 10 are disposed in a position dented from the region in which the absorber 4 is disposed to the non-skin-side only by a thickness part of the absorber 4, the opportunity for the skin-side embossed part 10 to be brought into contact with the skin surface is further reduced, and the skin contact can be further made to be soft.

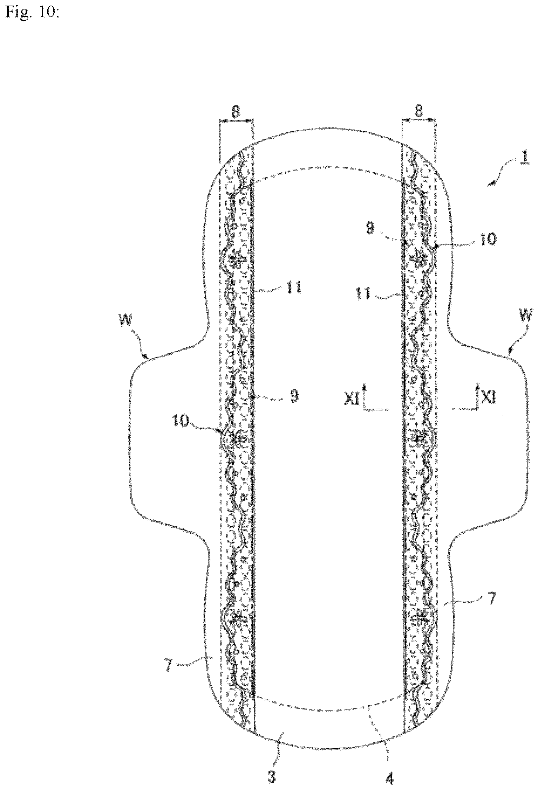

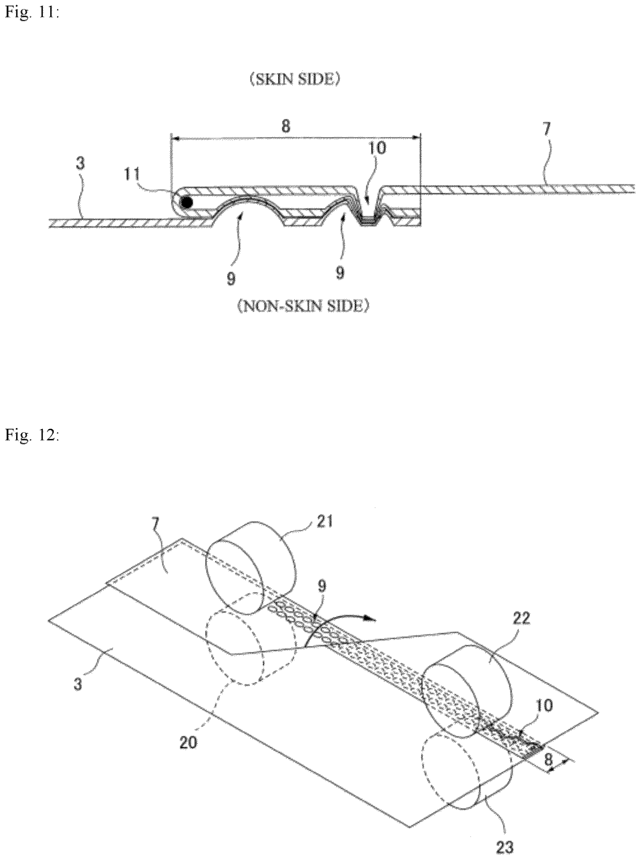

By the way, as shown in FIGs. 10 and 11, an elastically stretchable member 11 may be disposed among a plurality layers of laminated side sheet 7 in a streaked state along the longitudinal direction of the sanitary napkin 1, thereby making the sanitary napkin 1 to be easily curved. In this case, the elastically stretchable member 11 is preferably disposed to the inner side in the width direction from the lower layer side embossed portions 9 and the skin-side embossed portions 10. Thus, since the shrinkage force of the elastically stretchable member 11 easily acts, and the sanitary napkin 1 is easily curved in the longitudinal direction. In addition, the inner side portion in the width direction of the side sheet 7 in which the elastically stretchable member 11 is positioned stands up toward the skin-side, and easily acts as a barrier of the body fluid flowing in the side portion of the top-surface sheet 3, and thus, it is possible to enhance the effect of preventing side leakage of the body fluid. As the elastically stretchable member 11, a rubber thread is preferably used.

Next, in a method for manufacturing the sanitary napkin 1, a method for joining the top-surface sheet 3 and the side sheet 7 is described. As shown in FIG. 12, the side sheet 7 is laminated to the top-surface sheet 3 with the side edges substantially aligned to the skin-contact surface side of the top-surface sheet 3, and the laminate is introduced between a convex roll 20 having a large number of convex portions corresponding to the lower layer side embossed portions 9 on a circumferential surface of the convex roll 20 and a concave roll 21 having a large number of concave portions corresponding to the convex portions on a circumferential surface of the roll 21, and thus the lower layer side embossed portions 9 are added. Next, by folding back the side sheet 7 extending toward the inner side in the width direction from the lower layer side embossed portions 9 with the side edge of the inner side in the width direction of the lower layer side embossed portions 9 as a base end, and thereby the skin-contact surface side of the lower layer side embossed portions 9 is covered with this folded side sheet 7, and the laminated portion 8 of the side sheet 7 is formed. The laminate of the top-surface sheet 3 and the side sheet 7 in this state is introduced between the convex roll 22 having convex portions corresponding to the skin-side embossed portions 10 on the circumferential surface and the flat roll 23 having a flat circumferential surface, and pressed from the skin-contact surface side of the laminated portion 8 of the side sheet 7 to form the skin-side embossed portions 10 in which the side sheet 7 and the top-surface sheet 3 are integrally pressed. As mentioned above, joining of the top-surface sheet 3 and the side sheet 7 is completed.

Note here that in the above embodiment example, the lower layer side embossed portions 9 are formed in a shape in which the top-surface sheet 3 and the side sheet 7 are allowed to integrally bulge toward the skin-contact surface side or the non-skin-contact surface side by allowing the lower layer side embossed portions 9 between the convex roll 20 and the concave roller 21, but may be formed in a shape dented within a range of a thickness of a laminate of the top-surface sheet 3 and the side sheet 7 by allowing the lower layer side embossed portions 9 to pass between a convex roll and a flat roll, and pressing from the non-skin-contact surface side of the top-surface sheet 3 or the skin-contact surface side of the side sheet 7. Furthermore, in the above embodiment example, the skin-side embossed portions 10 are formed in a shape dented within a range of a thickness of a laminate of the top-surface sheet 3 and the folded side sheet 7 by allowing the skin-side embossed portions 10 to pass between the convex roll 22 and the flat roll 23, and pressing from the skin-contact surface side of the top-surface sheet 3, but may be formed in a shape in which the top-surface sheet 3 and the folded side sheet 7 are allowed to integrally bulge toward the non-skin-contact surface by allowing the skin-side embossed portions 10 to pass between the convex roll and a flat roll.

### [Other Embodiment Example]

In the above embodiment example, the laminated portion 8 of the side sheet 7 is formed of two layers, but may be formed of three or more layers. When the laminated portion 8 is formed of three or more layers, the lower layer side embossed portions 9 may join at least the bottom layer of the side sheet 7 and the top-surface sheet 3, and may integrally join the bottom layer of the side sheet 7 or the layer above it to the top-surface sheet 3. In other words, it is only required that the lower layer side embossed portions 9 are prevented from being applied to the top layer of the side sheet 7. When the laminated portion 8 is formed of three or more layers, a thickness of the laminated portion 8 is increased, thus improving an effect of both side portions of the top-surface sheet 3 as a barrier.

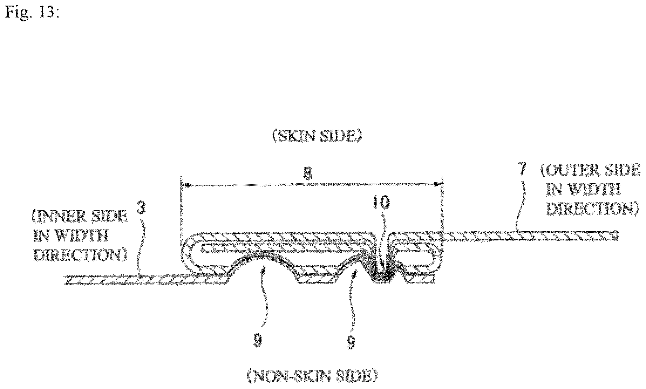

Furthermore, when, for example, the laminated portion 8 is formed of three layers, as shown in FIG. 13, it is preferable that folding is carried out such that the side edge of the side sheet 7 is not exposed to the inner side in the width direction, thereby preventing the wearing feeling from being deteriorated because the side edge is brought into contact with the skin surface. In the embodiment example shown in FIG. 13, the lower layer side embossed portions 9 are formed in the middle in the width direction of side sheet 7, and then side sheet 7 extending to the outer side in the width direction from the lower layer side embossed portions 9 is folded back toward the skin-contact surface side of the lower layer side embossed portions 9, and the side sheet 7 extending to the inner side in the width direction than the lower layer side embossed portions 9 is folded back toward the skin-surface side so as to wind the side edge of the folded side sheet 7, thus forming the laminated portion 8 having a three-layered structure.

### Reference Signs List

1... sanitary napkin, 2...back-surface sheet, 3...top-surface sheet, 4... absorber, 5...encapsulation sheet, 7... side sheet, 8...laminated portion, 9...lower layer side embossed portion, 10... skin-side embossed portion, 11... elastically stretchable member

## Claims

1. An absorbent article (1) comprising a top-surface sheet (3), a back-surface sheet (2), an absorber (4) interposed between the top-surface sheet (3) and the back-surface sheet (2), and a pair of left and right side sheets (7) over an entire length on both side portions of a skin-contact surface side,
wherein an inner side in a width direction of each of the side sheets (7) is folded back in the width direction to be laminated in a plurality of layers, and a laminated portion (8) of the side sheet includes:
lower layer side embossed portions (9) for joining a bottom layer of the side sheet (7) and the top-surface sheet (3) to each other, wherein at the skin-contact surface side of the lower layer side embossed portions (9) a sheet portion in the laminated portion (8) of the side sheet (7) in which the lower layer side embossed portions (9) are not provided, is laminated, whereby the lower layer side embossed portions (9) are configured not to be brought into direct contact with the skin when wearing;
and a skin-side embossed portions (10) obtained by integrally denting the side sheets (7) of the laminated portion (8) from the skin-contact surface side.

2. The absorbent article (1) according to claim 1, wherein the lower layer side embossed portions (9) and the skin-side embossed portions (10) are formed in different plane patterns.

3. The absorbent article (1) according to claim 1 or 2, wherein the skin-side embossed portions (10) are formed to have a smaller area of the embossed portions per unit area than the lower layer side embossed portions (9).

4. The absorbent article (1) according to any one of claims 1 to 3, wherein the lower layer side embossed portions (9) are formed in an intermittent plane pattern in which a pressed portion and a non-pressed portion are alternately disposed along a longitudinal direction of the absorbent article (1).

5. The absorbent article (1) according to any one of claims 1 to 4, wherein the lower layer side embossed portions (9) and the skin-side embossed portions (10) are formed in regions overlapping with each other in a thickness direction.

6. The absorbent article (1) according to any one of claims 1 to 4, wherein the lower layer side embossed portions (9) are formed in a region at an inner side in the width direction, and the skin-side embossed portions (10) are formed in a region at an outer side in the width direction, and thereby the lower layer side embossed portions (9) and the skin-side embossed portions (10) are formed in regions that do not overlap with each other in the thickness direction.

7. The absorbent article (1) according to any one of claims 1 to 6, wherein each of the side sheets (7) is disposed so as to extend toward the inner side portion in the width direction in a state in which one side portion is substantially aligned to the side portion of the top-surface sheet (3), and is folded back toward the skin-contact surface side from the lower layer side embossed portions (9) as a base such that another side portion extends to the side portion of the absorbent article (1).

8. The absorbent article (1) according to any one of claims 1 to 7, wherein an elastically stretchable member (11) is disposed between the layers of the side sheets (7) laminated in a plurality of layers along the longitudinal direction of the absorbent article (1), and the elastically stretchable member (11) is disposed at the inner side in the width direction from the lower layer side embossed portions (9) and the skin-side embossed portions (10).

9. The absorbent article (1) according to any one of claims 1 to 8, wherein the lower layer side embossed portions (9) are formed to have a sectional shape bulging toward a skin-contact surface side.

## Patentansprüche

1. Absorbierender Artikel (1), der eine oberseitige Oberflächenschicht (3), eine unterseitige Oberflächenschicht (2), einen Absorber (4), der zwischen der oberseitigen Oberflächenschicht (3) und der unterseitigen Oberflächenschicht (2) angeordnet ist, und ein Paar linker und rechter Seitenschichten (7) über eine gesamte Länge auf beiden Seitenabschnitten einer Hautkontaktoberflächenseite umfasst,
wobei eine Innenseite in einer Breitenrichtung jeder der Seitenschichten (7) in der Breitenrichtung zurückgefaltet ist, um in einer Vielzahl von Schichten laminiert zu werden, und wobei ein laminierter Abschnitt (8) der Seitenschicht folgendes umfasst:
auf der Seite einer unteren Schicht befindliche geprägte Abschnitte (9) zum miteinander Verbinden einer Bodenschicht der Seitenschicht (7) und der oberseitigen Oberflächenschicht (3), wobei an der Hautkontaktoberflächenseite der auf der Seite der unteren Schicht befindlichen geprägten Abschnitte (9) ein Schichtbereich in dem laminierten Abschnitt (8) der Seitenschicht (7), in dem die auf der Seite der unteren Schicht befindlichen geprägten Abschnitte (9) nicht vorgesehen sind, laminiert ist, wobei die auf der Seite der unteren Schicht befindlichen geprägten Abschnitte (9) konfiguriert sind, beim Tragen nicht in direkten Kontakt mit der Haut gebracht zu werden,
und geprägte Abschnitte (10) auf einer Hautseite, die durch integrales Eindrücken der Seitenschichten (7) des laminierten Abschnitts (8) von der Hautkontaktoberflächenseite her erhalten werden.

2. Absorbierender Artikel (1) gemäß Anspruch 1, wobei die auf der Seite der unteren Schicht befindlichen geprägten Abschnitte (9) und die geprägten Abschnitte (10) auf der Hautseite in unterschiedlichen ebenen Mustern ausgebildet sind.

3. Absorbierender Artikel (1) gemäß Anspruch 1 oder 2, wobei die geprägten Abschnitte (10) auf der Hautseite so geformt sind, dass sie pro Flächeneinheit eine kleinere Fläche der geprägten Abschnitte als die auf der Seite der unteren Schicht befindlichen geprägten Abschnitte (9) aufweisen.

4. Absorbierender Artikel (1) gemäß einem der Ansprüche 1 bis 3, wobei die auf der Seite der unteren Schicht befindlichen geprägten Abschnitte (9) in einem unterbrochenen ebenen Muster ausgebildet sind, in dem ein gepresster Abschnitt und ein nicht gepresster Abschnitt abwechselnd entlang einer Längsrichtung des absorbierenden Artikels (1) angeordnet sind.

5. Absorbierender Artikel (1) gemäß einem der Ansprüche 1 bis 4, wobei die auf der Seite der unteren Schicht befindlichen geprägten Abschnitte (9) und die geprägten Abschnitte (10) auf der Hautseite in Bereichen ausgebildet sind, die einander in einer Dickenrichtung überlappen.

6. Absorbierender Artikel (1) gemäß einem der Ansprüche 1 bis 4, wobei die auf der Seite der unteren Schicht befindlichen geprägten Abschnitte (9) in einem Bereich an einer Innenseite in der Breitenrichtung gebildet sind und die geprägten Abschnitte (10) auf der Hautseite in einem Bereich an einer Außenseite in der Breitenrichtung gebildet sind und dadurch die auf der Seite der unteren Schicht befindlichen geprägten Abschnitte (9) und die geprägten Abschnitte (10) auf der Hautseite in Bereichen gebildet sind, die einander in der Dickenrichtung nicht überlappen.

7. Absorbierender Artikel (1) gemäß einem der Ansprüche 1 bis 6, wobei jede der Seitenschichten (7) so angeordnet ist, dass sie sich in der Breitenrichtung in Richtung des inneren Seitenabschnitts in einem Zustand erstreckt, in dem ein Seitenabschnitt im Wesentlichen mit dem Seitenabschnitt der oberseitigen Oberflächenschicht (3) ausgerichtet ist, und von den auf der Seite der unteren Schicht befindlichen geprägten Abschnitten (9) als Basis zurück zu der Hautkontaktoberflächenseite gefaltet ist, so dass sich ein weiterer Seitenabschnitt bis zu dem Seitenabschnitt des absorbierenden Artikels (1) erstreckt.

8. Absorbierender Artikel (1) gemäß einem der Ansprüche 1 bis 7, wobei ein elastisch dehnbares Element (11) zwischen den Schichten der Seitenschichten (7), die in einer Vielzahl von Schichten entlang der Längsrichtung des absorbierenden Artikels (1) laminiert sind, angeordnet ist und das elastisch dehnbare Element (11) an der Innenseite in der Breitenrichtung von den auf der Seite der unteren Schicht befindlichen geprägten Abschnitten (9) und den geprägten Abschnitten (10) auf der Hautseite angeordnet ist.

9. Absorbierender Artikel (1) gemäß einem der Ansprüche 1 bis 8, wobei die auf der Seite der unteren Schicht befindlichen geprägten Abschnitte (9) so geformt sind, dass sie eine Querschnittsform aufweisen, die sich in Richtung einer Hautkontaktoberflächenseite wölbt.

## Revendications

1. Article absorbant (1) comprenant une feuille de surface supérieure (3), une feuille de surface arrière (2), un absorbeur (4) interposé entre la feuille de surface supérieure (3) et la feuille de surface arrière (2), et une paire de feuilles latérales gauche et droite (7) sur une longueur entière sur les deux parties latérales d'un côté de surface de contact avec la peau,
dans lequel un côté intérieur dans une direction de largeur de chacune des feuilles latérales (7) est replié dans la direction de largeur pour être stratifié dans une pluralité de couches, et une partie stratifiée (8) de la feuille latérale inclut :
des parties gaufrées côté couche inférieure (9) permettant d'assembler entre elles une couche du bas de la feuille latérale (7) et la feuille de surface supérieure (3), dans lequel, sur le côté de surface de contact avec la peau des parties gaufrées côté couche inférieure (9), une partie de feuille dans la partie stratifiée (8) de la feuille latérale (7) dans laquelle les parties gaufrées côté couche inférieure (9) ne sont pas présentes est stratifiée, moyennant quoi les parties gaufrées côté couche inférieure (9) sont configurées pour ne pas être mises en contact direct avec la peau pendant l'utilisation ;
et des parties gaufrées côté peau (10) obtenues par indentation intégrale des feuilles latérales (7) de la partie stratifiée (8) à partir du côté de surface de contact avec la peau.

2. Article absorbant (1) selon la revendication 1, dans lequel les parties gaufrées côté couche inférieure (9) et les parties gaufrées côté peau (10) sont formées dans des différents motifs plans.

3. Article absorbant (1) selon la revendication 1 ou 2, dans lequel les parties gaufrées côté peau (10) sont formées de manière à présenter une plus petite aire des parties gaufrées par unité d'aire que les parties gaufrées côté couche inférieure (9).

4. Article absorbant (1) selon l'une quelconque des revendications 1 à 3, dans lequel les parties gaufrées côté couche inférieure (9) sont formées dans un motif plan intermittent dans lequel une partie comprimée et une partie non-comprimée sont disposées alternativement le long d'une direction longitudinale de l'article absorbant (1).

5. Article absorbant (1) selon l'une quelconque des revendications 1 à 4, dans lequel les parties gaufrées côté couche inférieure (9) et les parties gaufrées côté peau (10) sont formées dans des régions qui se chevauchent les unes les autres dans un sens de l'épaisseur.

6. Article absorbant (1) selon l'une quelconque des revendications 1 à 4, dans lequel les parties gaufrées côté couche inférieure (9) sont formées dans une région sur un côté intérieur dans la direction de largeur, et les parties gaufrées côté peau (10) sont formées dans une région sur un côté extérieur dans la direction de largeur, et les parties gaufrées côté couche inférieure (9) et les parties gaufrées côté peau (10) sont formées dans des régions qui ne se chevauchent pas les unes les autres dans le sens de l'épaisseur.

7. Article absorbant (1) selon l'une quelconque des revendications 1 à 6, dans lequel chacune des feuilles latérales (7) est disposée de manière à s'étendre vers la partie latérale intérieure dans la direction de largeur dans un état dans lequel une partie latérale est substantiellement alignée par rapport à la partie latérale de la feuille de surface supérieure (3), et est repliée vers le côté de surface de contact avec la peau à partir des parties gaufrées côté couche inférieure (9) comme base, de sorte qu'une autre partie latérale s'étend vers la partie latérale de l'article absorbant (1).

8. Article absorbant (1) selon l'une quelconque des revendications 1 à 7, dans lequel un élément étirable élastiquement (11) est disposé entre les couches des feuilles latérales (7) stratifiées dans une pluralité de couches le long de la direction longitudinale de l'article absorbant (1), et l'élément étirable élastiquement (11) est disposé sur le côté intérieur dans la direction de largeur à partir des parties gaufrées côté couche inférieure (9) et des parties gaufrées côté peau (10).

9. Article absorbant (1) selon l'une quelconque des revendications 1 à 8, dans lequel les parties gaufrées côté couche inférieure (9) sont formées de manière à présenter une forme sectionnelle bombée vers un côté de surface de contact avec la peau.
